# EUROPEAN PATENT APPLICATION

(11) **EP 1 925 316 A2**
(43) Date of publication of application: **28.05.2008**
(21) Application number: 07108795.1
(22) Date of filing: 06.09.2002
(51) Int. Cl.: A61K 38/22, A61P 15/00, A61P 15/02

(54) **Compositions for treating vaginal, cervical and uterine epithelial lesions**

(30) Priority: 06.09.2001 US 317657 P; 05.09.2002 US 235238
(62) Divisional of application: 02757620.6
(71) Applicant: THE GENERAL HOSPITAL CORPORATION, Boston, MA 02114 (US)
(72) Inventor: Podolsky, Daniel, K., Wellesley, MA 02481 (US)
(74) Representative: Gervasi, Gemma

(57) **Abstract**

This invention features the use of human intestinal trefoil peptide for the manufacture of a medicament for the treatment or prevention of lesions of the vaginal, cervical, or uterine epithelium of a mammal. The intestinal trefoil peptide can be administered either alone or in combination with one or therapeutic agents. According to a preferred embodiment said intestinal trefoil peptide is spasmolytic polypeptide, pS2, intestinal trefoil factor (ITF), ITF₁₅₋₇₃, ITF₂₁₋₇₃, ITF₁₋₇₂, ITF₁₅₋₇₂, or ITF₂₁₋₇₂.

## Description

### Field of the Invention

This invention relates to methods and compositions for treating and preventing vaginal, cervical, or uterine epithelial lesions in a mammal that can result from a variety of natural and iatrogenic causes.

### Background of the Invention

Vaginal epithelial lesions can be caused by a variety of conditions, including sexual intercourse, childbirth, infection, and surgical procedures. Consequently, the magnitude of these lesions can range from small cuts and abrasions to major tears that are sometimes associated with vaginal childbirth. Lesions can be focal, as with a surgical procedure such as episiotomy, or more diffuse such as the wider irritation caused by a severe yeast (Candida *spp*.) infection. Vaginal epithelial destruction can also be a consequence of cancer chemotherapy or radiotherapy of pelvic structures.

Injury to the vaginal epithelium can pose a significant health risk and can result in permanent alterations to reproductive organs. Vaginal lesions can result in scarring or physical discomfort and may provide a site for an opportunistic infection. The susceptibility of the epithelial layer to physical or microbial damage can be exacerbated by post-menopausal conditions like atrophic vaginitis which is characterized by a thinning of the epithelium. Symptoms of such lesions range from mild irritation to severe pain with bleeding. Currently, there is a dearth of medications available for the treatment of vaginal epithelial injury.

### Summary of the Invention

This invention features methods and compositions for treating or preventing epithelial lesions of the vagina, cervix, or uterus in a mammal by intravaginal or intrauterine administration of a therapeutically effect amount of an intestinal trefoil peptide. Treatment or prevention of lesions according to the invention can speed healing, reduce pain, delay or prevent occurrence of the lesion, and inhibit expansion, secondary infection, or other complications of the lesion. Preferably, the mammal is a human. In particularly useful embodiments, the intestinal trefoil peptide is SP, pS2, ITF, ITF₁₅₋₇₃, ITF₂₁₋₇₃, ITF₁₋₇₂, ITF₁₅₋₇₂, or ITF₂₁₋₇₂, and is present in a pharmaceutical composition containing a pharmaceutically acceptable carrier. Other useful intestinal trefoil peptides include polypeptides that are substantially identical to SP, pS2, ITF, ITF₁₅₋₇₃, ITF₂₁₋₇₃, ITF₁₅₋₇₂, or ITF₂₁₋₇₂. Preferably, the intestinal trefoil peptide is ITF, which may be administered as a monomer, a dimer, or another multimeric form.

According to the invention, the lesion that is treated or prevented can result from, for example, a microbial (e.g., bacterial, viral, or fungal) infection, antineoplastic chemotherapy or radiotherapy, an inflammatory or allergic reaction, or a lesion caused by a local biopsy, surgical procedure or other traumatic injury. Methods and compositions of the present invention are particularly useful for treating lesions caused by pelvic inflammatory disease (PID), bacterial infections by, for example, *N. gonorrhea, T. pallidum, Gardnerella spp., and Chlamydia spp.,* viral infections by, for example, Herpes Simplex virus and papilloma virus, or fungal infections by, for example, *Candida albicans.* Traumatic lesions of the vagina, cervix, or uterus that are amenable to treatment include, for example, those caused by a surgical procedure (e.g., episiotomy) or childbirth. Uterine lesions caused by the excision of uterine fibroids or polyps are particularly amenable to treatment with an intestinal trefoil peptide according to this invention. The methods and compositions of this invention are also useful for treating patients having atrophic vaginal mucosa; a condition frequently associated with menopause.

In preferred embodiments of the methods and compositions, a second therapeutic agent is included. Desirable second therapeutic agents include anti-inflammatory agents, antibacterial agents such as, generally, penicillins, cephalosporins, tetracyclines, and aminoglycosides, (e.g., azithromycin, doxycycline, erythromycin, spectinomycin, metronidazole, ceftizoxime, cefotaxime, cefixime, ceftriaxone, cirpofloxacin, ofloxacin, and levofloxacin) antifungal agents (e.g., nystatin, butoconazole, clotrimazole, enilconazole, itraconazole, ketoconazole, miconazole, and amphotericin B), antiviral agents (e.g., acyclovir), analgesics (e.g., lidocaine or benzocaine), or steroids (e.g., triamcinolone, budesonide, hydrocortisone, estrogen, and estradiol). The second therapeutic agent may also be an agent that reverses a pH change in the vaginal or uterus that is caused by a microbial infection. The second therapeutic agent may be administered within (either before or after administration of the intestinal trefoil peptide) 14 days, 7 days, 1 day, 12 hours, 1 hour, or simultaneously with the intestinal trefoil peptide.

The second therapeutic agent can be present in the same or different pharmaceutical compositions as the intestinal trefoil peptide. When the second therapeutic agent is present in a different pharmaceutical composition, different routes of administration may be used. For example, the second therapeutic may be administered orally, or by intravenous, intramuscular, or subcutaneous injection. The second therapeutic need not be administered intravaginally. Frequently, for treating certain vaginal infections and sexually transmitted diseases, an antibiotic is administered orally and the intestinal trefoil peptide is administered intravaginally.

Suitable pharmaceutical compositions are formulated as a vaginal suppository, an aqueous rinse, a cream, or a gel, and include at least an intestinal trefoil peptide and a pharmaceutically acceptable carrier. Treatment using trefoil peptide-containing compositions of this invention is typically self-administered. However, trefoil peptide therapy may be administered by a medical professional or other health care provider. In particular, trefoil peptide therapy of uterine lesions may be administered by a medical professional. For example, a trefoil peptide-containing gel, cream, or bioerodable polymer may be applied to lesions caused by the removal of uterine fibroids or polyps immediately after the surgical or laser removal procedure. Of course, pharmaceutical compositions may contain two, three, or more intestinal trefoil peptides. Particularly useful pharmaceutical compositions also contain a mucoadhesive or viscosity-enhancing agent.

Mammalian intestinal trefoil peptides were discovered in 1982. One of the mammalian intestinal trefoil peptides, human intestinal trefoil factor (ITF; TFF3), has been characterized extensively, and is described in U.S. Patent Nos. 6,063,755, and 6,221,840, ' The other two known human intestinal trefoil peptides are spasmolytic polypeptide (SP; TFF2) and pS2 (TFF1). Intestinal trefoil peptides, described extensively in the literature (e.g., Sands et al., Annu. Rev. Physiol. 58: 253-273, 1996), are expressed in the gastrointestinal tract and have a three-loop structure formed by intrachain disulfide bonds between conserved cysteine residues. These peptides protect the intestinal tract from injury and can be used to treat intestinal tract disorders such as peptic ulcers and inflammatory bowel disease. Homologs of these human peptides have been found in a number of non-human animal species. All members of this protein family, both human and non-human, are referred to herein as intestinal trefoil peptides. Human ITF will be referred to most extensively in this application; however, the activity of human ITF is common to each of the mammalian intestinal trefoil peptides.

"Intestinal trefoil peptide," as used herein, includes all mammalian homologs of human spasmolytic polypeptide (SP; also known as TFF2), human pS2 (also known as TFF1) and human intestinal trefoil factor (ITF; also known as TFF3), and biologically active fragments thereof. Homologs of the trefoil peptides have, preferably, 70% amino acid identity to the human sequence, more preferably 85% identity, most preferably 95%, or even 99% sequence identity. The length of comparison sequences will generally be at least about 10 amino acid residues, usually at least 20 amino acid residues, more usually at least 30 amino acid residues, typically at least 45 amino acid residues, and preferably more than 60 amino acid residues. Alternatively, intestinal trefoil peptides are polypeptides encoded by a polynucleotide that hybridizes with high stringency to the human ITF, pS2, or SP cDNAs provided in SEQ ID NOs: 4, 5, and 6, respectively, or the human ITF, pS2, or SP genes provided in SEQ ID NOs: 7, 8, and 9, respectively.

The term "fragment" is meant to include polypeptides that are truncations or deletions of SP, pS2 and ITF. Preferably, the fragments have 70% amino acid identity to the corresponding regions of the human polypeptide sequence. More preferably, the fragments are 85% identical, most preferably 95%, or even 99% identical to the human polypeptide sequence to which they correspond. The length of comparison sequences will generally be at least about 10 amino acid residues, usually at least 20 amino acid residues, more usually at least 30 amino acid residues, typically at least 45 amino acid residues, and preferably more than 60 amino acid residues.

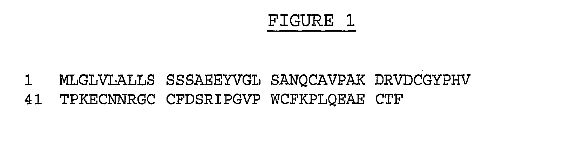

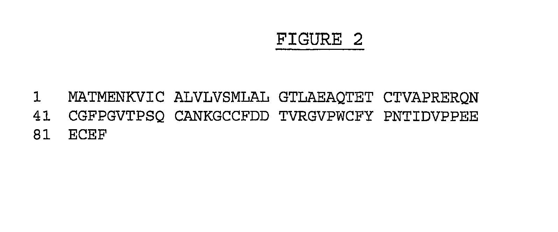

Preferable fragments contain four cysteine residues in any positions which correspond to the cysteines at positions 25, 35, 45, 50, 51, 62, or 71, of human ITF (Figure 1), or positions 31, 41, 51, 56, 57, 68, and 82 of human pS2 (Figure 2). More preferably, fragments contain five cysteine residues at these positions. Most preferably, six, or even all seven cysteines are present.

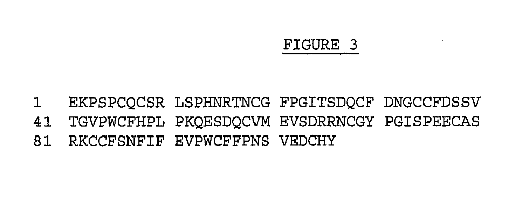

Fragments of SP are meant to include truncations or deletions and preferably have 70% sequence identity to the corresponding human SP polypeptide sequence (Figure 3). More preferably, the fragments are 85% identical, most preferably 95%, or even 99% identical to the human polypeptide sequence. Preferably, active fragments contain at least four cysteine residues which correspond to positions 6, 8, 19, 29, 34, 35, 46, 58, 68, 78, 83, 84, 95, and 104 in the human SP polypeptide. More preferably, fragments contain six cysteines which correspond to these positions. Even more preferable are fragments that contain eight cysteines. Most preferable are fragments that contain cysteines at ten, twelve, or even, all fourteen positions.

It is recognized in the art that one function of the identified cysteine residues is to impart the characteristic three-loop (trefoil) structure to the protein. Accordingly, preferred fragments of ITF and pS2 have a least one loop structure, more preferably, the fragments have two loop structures, and most preferably, they have three loop structures. It is equally well recognized that the native SP polypeptide has a six loop confirmation. Preferable fragments contain at least two of these loop structures, more preferably, four loop structures are conserved, and most preferably, five, or even all six loop structures are present.

By "co-formulated" is meant any single pharmaceutical composition which contains two or more therapeutic or biologically active agents.

By "pharmaceutical preparation" or "pharmaceutical composition" is meant any composition which contains at least one therapeutically or biologically active agent and is suitable for administration to a patient. For the purposes of this invention, pharmaceutical compositions suitable for delivering a therapeutic to the vagina, cervix, or uterus include, but are not limited to suppositories, rinses (e.g., douches), pastes, creams, gels, and bioerodable matrices. Any of these formulations can be prepared by well known and accepted methods of art. See, for example, Remingtion: The Science and Practice of Pharmacy, 19th edition, (ed. AR Gennaro), Mack Publishing Co., Easton, PA, 1995.

By "therapeutically effective amount" is meant an amount sufficient to provide medical benefit. When administering trefoil peptides to a human patient according to the methods described herein, a therapeutically effective amount is usually about 1-2500 mg of intestinal trefoil peptide per dose. Preferably, the patient receives, 10 mg, 100 mg, 500 mg, 750 mg,1000 mg, 1500 mg, or 2000 mg of intestinal trefoil peptide in each dose. Dosing is typically performed 1-5 times each day.

By "vagina" is meant the internal structures of the female reproductive tract extending from the cervix of the uterus to the vestibule. As used herein, the term "vagina" also includes the external genitalia (i.e., labia majora, labia minora, and clitoris).

By "biologically active," when referring to an intestinal trefoil peptide, fragment, or homolog is meant any polypeptide that exhibits an activity common to its related, naturally occurring family member, and that the activity is common to the family of naturally occurring intestinal trefoil peptides. An example of a biological activity common to the family of trefoil peptides is the ability to alter gastrointestinal motility in a mammal.

By "isolated DNA" is meant DNA that is free of the genes which, in the naturally-occurring genome of the organism from which the given DNA is derived, flank the DNA. Thus, the term "isolated DNA" encompasses, for example, cDNA, cloned genomic DNA, and synthetic DNA.

By "treating" is meant administering a pharmaceutical composition for prophylactic and/or therapeutic purposes. The active ingredients of the pharmaceutical composition can treat the primary indication (e.g., epithelial lesion) or secondary symptoms (e.g., concomitant infection, pain, or inflammation).

By "analgesic" is meant an agent which relieves pain by elevating the pain threshold without significantly disturbing the consciousness of the patient.

By "antimicrobial agent" is meant any compound that alters the growth of bacteria or fungi cells, or viruses whereby growth is prevented, stabilized, or inhibited, or wherein the microbes are killed. In other words, the antimicrobial agents can be microbiocidal or microbiostatic.

By "antineoplastic therapy" is meant any treatment regimen used to treat cancer. Typical antineoplastic therapies include chemotherapy and radiation therapy.

By "substantially identical" is meant a polypeptide or nucleic acid exhibiting at least 75%, but preferably 85%, more preferably 90%, most preferably 95%, or 99% identity to a reference amino acid or nucleic acid sequence . For polypeptides, the length of comparison sequences will generally be at least 20 amino acids, preferably at least 30 amino acids, more preferably at least 40 amino acids, and most preferably 50 amino acids. For nucleic acids, the length of comparison sequences will generally be at least 60 nucleotides, preferably at least 90 nucleotides, and more preferably at least 120 nucleotides.

By "high stringency conditions" is meant any set of conditions that are characterized by high temperature and low ionic strength and allow hybridization comparable with those resulting from the use of a DNA probe of at least 40 nucleotides in length, in a buffer containing 0.5 M NaHP04, pH 7.2, 7% SDS, 1mM EDTA, and 1% BSA (Fraction V), at a temperature of 65 C, or a buffer containing 48% formamide, 4.8X SSC, 0.2 M Tris-Cl, pH 7.6, 1X Denhardt's solution, 10% dextran sulfate, and 0.1% SDS, at a temperature of 42°C. Other conditions for high stringency hybridization, such as for PCR, Northern, Southern, or in situ hybridization, DNA sequencing, etc., are well-known by those skilled in the art of molecular biology. See, e.g., F. Ausubel et al., Current Protocols in Molecular Biology, John Wiley & Sons, New York, NY, 1998,

Other features and advantages of the invention will be apparent from the following detailed description, and from the claims.

### Brief Description of the Drawings

Figure 1 is an amino acid sequence of a human intestinal trefoil factor (ITF; Accession No. BAA95531).
Figure 2 is an amino acid sequence of a human pS2 protein (Accession No. NP_003216).
Figure 3 is an amino acid sequence of human spasmolytic polypeptide (SP; Accession No. 1909187A).
Figure 4 is a cDNA sequence encoding a human intestinal trefoil factor.
Figure 5 is a cDNA sequence encoding a human pS2 protein.
Figure 6 is a cDNA sequence encoding a human spasmolytic polypeptide.
Figure 7 is the nucleotide sequence of a gene encoding human intestinal trefoil factor (locus 10280533:52117-55412).
Figure 8 is the nucleotide sequence of a gene encoding human pS2 protein (locus 10280533:16511-21132).
Figure 9 is the nucleotide sequence of a gene encoding human spasmolytic polypeptide (locus 10280533:957-5208).

### Detailed Description

The invention provides methods and compositions useful for the treatment of a wide range of lesions of the vagina and cervix. The vaginal and cervical epithelial lesions amenable to treatment according to the present invention can be induced by physical trauma, including those resulting from sexual intercourse, contact with a foreign object, surgical intervention (*e.g*., episiotomy, hysterectomy, biopsy), or vaginal childbirth. The methods of the present invention can also be used to promote epithelial healing and integrity following denudation or injury caused by an infection (*e.g*., viral, bacterial, fungal), systemic or local cancer chemotherapy, or pelvic radiotherapy.

### Pharmaceutical Preparations

### Vaginal Rinses

A vaginal rinse, or douche, is used to deliver the intestinal trefoil peptides to the cells of the vagina and cervix. Douche volumes of about 50-300 ml can be used.

| *ITF-containing Douche for Treating Minor Vaginal Irritation* | |
|---|---|
| Intestinal trefoil factor | 500 mg |
| Povidone-iodine | 0.30 % |
| Distilled water | 150 ml |

### Vaginal Suppositories and Pessaries

Suppositories are solid dosage forms for insertion into the vagina for delivering medication to the vagina, cervix, and uterus. Typically, after insertion, the suppository softens, melts, disperses, or dissolves. Vaginal suppositories are usually about 1-7 grams each and tapered on both ends. Either a fatty or a water soluble/water miscible suppository base can be used in the compositions of this invention. Suitable fatty bases include, for example, cocoa butter, starch, theobroma oil, vegetable oils modified by esterification, hydrogenation, glycerinated gelatin, and high molecular weight polyethylene glycols. Sustained release and/or prolonged contact of the therapeutics can be achieved by proper selection of a fatty suppository base material. Cocoa butter, for example, melts quickly at body temperature but is immiscible with body fluids, resulting in a prolonged but low level delivery of fat-soluble therapeutics to the affected sites. Alternatively, water soluble or water miscible bases (e.g., polyethylene glycols and glycol-surfactant mixtures) typically dissolve or disperse quickly, resulting in a rapid delivery of the therapeutic to the affected sites. An exemplary suppository formulation is provided below.

| *ITF-containing Suppository Tablet* | |
|---|---|
| Intestinal trefoil factor | 300 mg |
| Polyethylene glycol 1000 | 96% |
| Polyethylene glycol 4000 | 4% |

This formulation has a low-melting point and may require refrigeration to maintain in a solid state. Because the intestinal trefoil peptides are proteinaceous, refrigeration may be desirable. The low melting point of the formulation results in rapid suppository melting following insertion, resulting in greater patient comfort. If refrigeration is not possible, or if heat molding techniques are used, the amount of polyethylene glycol 4000 may be increased to achieve a sufficiently heat stable formulation.

In an alternative formulation, the intestinal trefoil peptides and/or other therapeutics can be encapsulated in bioerodable microspheres rather than being dissolved in the aqueous phase of the formulation. A wide variety of microencapsulation drug delivery systems have been developed and many share similar polymeric compositions as used for bioerodable films. Polymers commonly used in the formation of microspheres include, for example, methylacrylate polymers, poly-ε-caprolactone, poly(ε-caprolactone-Co-DL-lactic acid), poly(DL-lactic acid), poly(DL-lactic acid-Co-glycolic acid) and poly(ε-caprolactone-Co-glycolic acid) (see, for example, Pitt et al., J. Pharm. Sci., 68:1534, 1979).

Microspheres can be made by procedures well known in the art including spray drying, coacervation, and emulsification (see for example Davis et al. Microsphere and Drug Therapy, Elsevier, 1984; Benoit et al. Biodegradable Microspheres: Advances in Production Technologies, Chapter 3, Ed. Benita, S, Dekker, New York, 1996; Microencapsulation and Related Drug Processes, Ed. Deasy, Dekker, 1984, New York; U.S. Patent No. 6,365,187). Preferably, the microspheres are bioadhesive or are prepared in formulations containing a bioadhesive excipient.

Other technical features of the intestinal trefoil peptide-containing solutions are easily modified to suit the specific pharmaceutical formulation and the clinical indication being treated. For example, the pH and osmolarity of the formulation may be adjusted to confer trefoil peptide stability, while minimizing vaginal and cervical irritancy and sensitivity.

### 0intments, Pastes, and Gels

Lesions of the vaginal and cervical epithelium and of the external genitalia and the surrounding skin are amenable to trefoil peptide therapy delivered as an ointment, paste, or gel. The viscous nature of these types of preparations allows for direct application into the wound site. Optionally, the wound site can be covered with a dressing to retain the trefoil peptide-containing composition, protect the lesion and/or absorb exudate. As discussed further below, these preparations are particularly useful to restore epithelial integrity following traumatic surgical procedures (e.g., episiotomy). Such viscous formulations may also have a local barrier effect thereby reducing irritation and pain. Alternatively, the ointment, paste, or gel composition may contain, in addition to a trefoil peptide, an antimicrobial such as an antifungal agent. These combinations are particularly useful for treating vaginal infections and certain sexually transmitted diseases.

| *ITF-containing Paste for Treating Candidosis* | |
|---|---|
| Intestinal trefoil factor | 500 mg |
| Tioconazole | 300 mg (6.5%) |
| White Petrolatum | 4.6 grams |

### Mucoadhesives

A mucoadhesive excipient can be added to any of the previously described pharmaceutical compositions. The mucoadhesive formulations coat the lesioned area, resulting in retention of the intestinal trefoil peptide at the lesion site, providing protection, inhibiting irritation, and accelerating healing of inflamed or damaged tissue. Mucoadhesive formulations suitable for use in these pharmaceutical preparations are well known in the art (e.g., U.S. Patent No. 5,458,879). Particularly useful mucoadhesives are hydrogels composed of about 0.05-20% of a water-soluble polymer such as, for example, poly(ethylene oxide), poly(ethylene glycol), poly(vinyl alcohol), poly(vinyl pyrrolidine), poly(acrylic acid), poly(hydroxy ethyl methacrylate), hydroxyethyl ethyl cellulose, hydroxy ethyl cellulose, chitosan, and mixtures thereof. These polymeric formulations can also contain a dispersant such as sodium carboxymethyl cellulose (0.5-5.0%).

Other preferred mucoadhesive excipients for liquid compositions are ones that allow the composition to be administered as a flowable liquid but will cause the composition to gel in the vagina, thereby providing a bioadhesive effect which acts to hold the therapeutic agents at the lesion site for an extended period of time. The anionic polysaccharides pectin and gellan are examples of materials which when formulated into a suitable composition will gel in the vagina, owing to the presence of cations in the mucosal fluids. The liquid compositions containing pectin or gellan will typically consist of 0.01-20% w/v of the pectin or gellan in water or an aqueous buffer system.

Other useful compositions which promote mucoadhesion and prolonged therapeutic retention in the vagina are colloidal dispersions containing 2-50% colloidal particles such as silica or titanium dioxide. Such formulations form as a flowable liquid with low viscosity suitable as vaginal rinse; however, the particles interact with glycoprotein, especially mucin, transforming the liquid into a viscous gel, providing effective mucoadhesion (e.g., U.S. Patent Nos. 5,993,846 and 6,319,513).

### Therapeutics Agents

### Trefoil Peptides

The therapeutic intestinal trefoil peptide(s) are typically mammalian intestinal trefoil peptides. Preferably, human intestinal trefoil peptides are used; however, trefoil peptides from other species including rat, mouse, and non-human primate, may be used. Typically, the intestinal trefoil peptide is intestinal trefoil factor (ITF); however, spasmolytic polypeptide (SP), or pS2 are also useful.

The intestinal trefoil peptides are administered at 1-5000 mg per dose, preferably 5-2500 mg per dose, or more preferably 10-1500 mg per dose, depending on the nature and condition of the lesion being treated, the anticipated frequency and duration of therapy, and the type of pharmaceutical composition used to deliver the trefoil peptide. The intestinal trefoil peptides are typically administered 1-5 times per day.

### Therapeutic Fragments of Intestinal Trefoil Factor (ITF)

We have also discovered that particular ITF fragments retain biological activity and may be substituted in any method or composition in which ITF is used. Methods and compositions containing ITF, in which these ITF fragments may be substituted, are described, for example, in U.S. Patent Nos. 6,063,755 and 6,221,840, and U.S. Patent Application Nos. 10/131,363, filed April 24, 2002, 60/317,657, filed September 6, 2001, 60/327,673, filed October 5, 2002, 60/333,836, filed November 28, 2001, and 60/367,574, filed March 26, 2002

Particularly useful ITF fragments that retain biological activity include the polypeptide corresponding to amino acid residues 15-73 of SEQ ID NO:1 1 (ITF₁₅₋₇₃) and amino acid residues 21-73 of SEQ ID NO:1 (ITF₂₁₋₇₃). Other useful ITF fragments are formed following cleavage of the C-terminal phenylalanine residue (i.e., ITF₁₋₇₂, ITF₁₅₋₇₂, and ITF₂₁₋₇₂).

The biologically active ITF fragments of this invention can be produced using any appropriate method. For example, cDNA encoding the desired ITF fragment can be used with any method known in the art for producing recombinant proteins. Exemplary methods are provided herein. ITF fragments, particularly ITF₂₁₋₇₃, can be produced using a *Pichia* yeast expression system (see, for example, U.S. Patent Nos. 4,882,279 and 5,122,465) transformed with a cDNA encoding long ITF species, such as the full length ITF (e.g., SEQ ID NO:4) or ITF₁₅₋₇₃, when the fermentation culture is maintained at pH ~ 5.0.

### Anti-Inflammatory Agents

Any suitable anti-inflammatory agent can be administered with the trefoil peptide and employed using the method of this invention, either in the same or different pharmaceutical compositions. Suitable anti-inflammatory agents include, but are not limited to non-steroidal anti-inflammatory drugs (*e*.*g*., ibuprofen, tacrolimus), cyclooxygenase-2-specific inhibitors such as rofecoxib (Vioxx®) and celecoxib (Celebrex®), topical glucocorticoid agents and specific cytokines directed at T lymphocyte function. Anti-inflammatory concentrations known to be effective for reducing vaginal or cervical inflammation can be used. For example, ibuprofen may be present in the composition at concentrations sufficient to deliver between 25-800 mg per day to the lesion. Corticosteroids may be co-formulated with a trefoil peptide at concentrations known to be effective for local intravaginal use.

### Antimicrobial Agents

Any of the many known antimicrobial agents can be administered with the trefoil peptide and employed using the method of this invention, either in the same or different pharmaceutical compositions. Antimicrobial agents include antibacterials, antifungals, and antivirals.

Examples of antibacterial agents (antibiotics) include the penicillins (e.g., penicillin G, ampicillin, methicillin, oxacillin, and amoxicillin), the cephalosporins (e.g., cefadroxil, ceforanid, cefotaxime, and ceftriaxone), the tetracyclines (e.g., doxycycline, minocycline, and tetracycline), the aminoglycosides (e.g., amikacin, gentamycin, kanamycin, neomycin, streptomycin, and tobramycin), the macrolides (e.g., azithromycin, clarithromycin, and erythromycin), the fluoroquinolones (e.g., ciprofloxacin, lomefloxacin, and norfloxacin), and other antibiotics including chloramphenicol, clindamycin, cycloserine, isoniazid, rifampin, and vancomycin.

Antiviral agents are substances capable of destroying or suppressing the replication of viruses. Examples of anti-viral agents include 1,-D-ribofuranosyl-1,2,4-triazole-3 carboxamide, 9->2-hydroxy-ethoxy methylguanine, adamantanamine, 5-iodo-2'-deoxyuridine, trifluorothymidine, interferon, adenine arabinoside, protease inhibitors, thymidine kinase inhibitors, sugar or glycoprotein synthesis inhibitors, structural protein synthesis inhibitors, attachment and adsorption inhibitors, and nucleoside analogues such as acyclovir, penciclovir, valacyclovir, and ganciclovir.

Antifungal agents include both fungicidal and fungistatic agents such as, for example, amphotericin B, butylparaben, clindamycin, econaxole, fluconazole, flucytosine, griseofulvin, nystatin, clotrimazole, ketoconazole, enilconazole, itraconazole, butoconazole, and miconazole.

### Analgesics and Anesthetics

Any of the commonly used topical analgesics can be used in the methods and compositions of the invention. The analgesic is present in an amount such that there is provided to the vaginal or cervical lesion a concentration of between one-half and five percent concentration for lidocaine (5-50 mg/ml in 20-40 ml per dose of liquid). Examples of other useful anesthetics include procaine, lidocaine, tetracaine, dibucaine, benzocaine, p-buthylaminobenzoic acid 2-(diethylamino) ethyl ester HC1, mepivacaine, piperocaine, and dyclonine.

Other analgesics that may be administered in different pharmaceutical compositions and by other routes include opioids such as, for example, morphine, codeine, hydrocodone, and oxycodone. Any of these analgesics may also be co-formulated with other compounds having analgesic or anti-inflammatory properties, such as acetaminophen, aspirin, and ibuprofen.

### Steroids

Steroids may be used to treat vaginal, cervical, or uterine lesions. Steroids suitable for formulation in a paste preparation include, for example, triamcinolone (0.1 %), hydrocortisone, fluticasone, budesonide, or beclomethasone. Estrogens (e.g., estradiol) are particularly useful for treating atrophic vaginal mucosa; a condition frequently associated with menopause.

### Production of Intestinal Trefoil Peptides

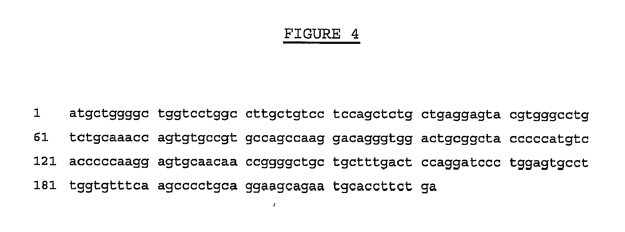

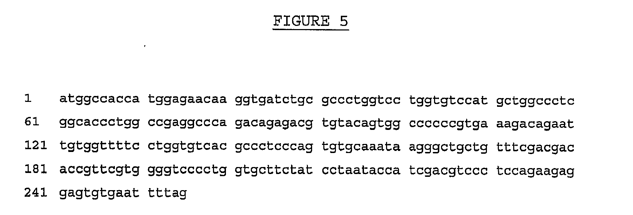

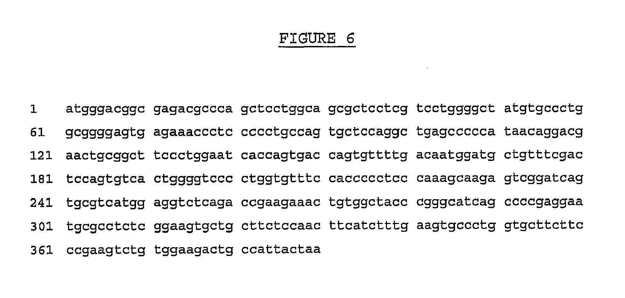

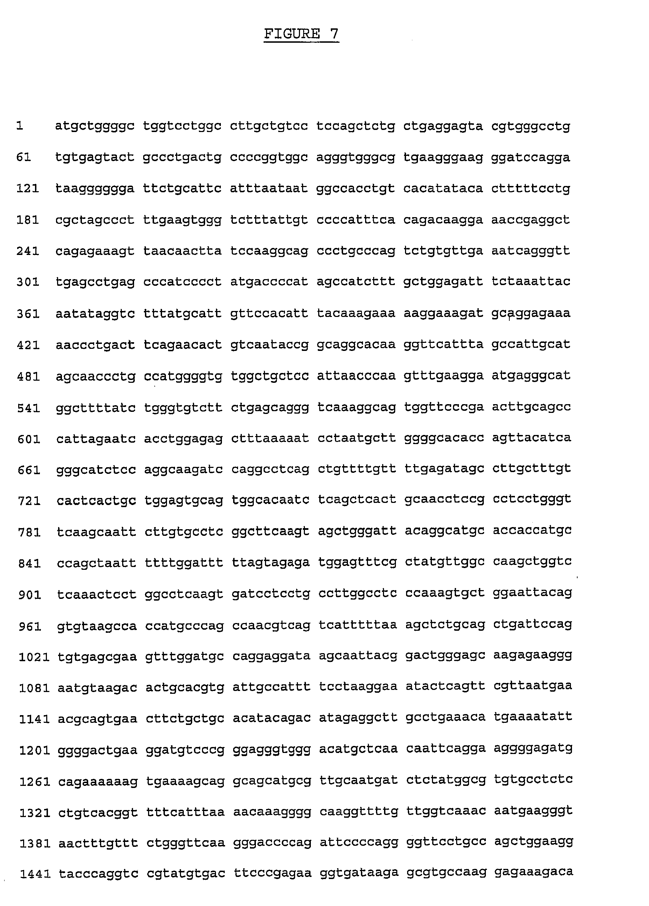

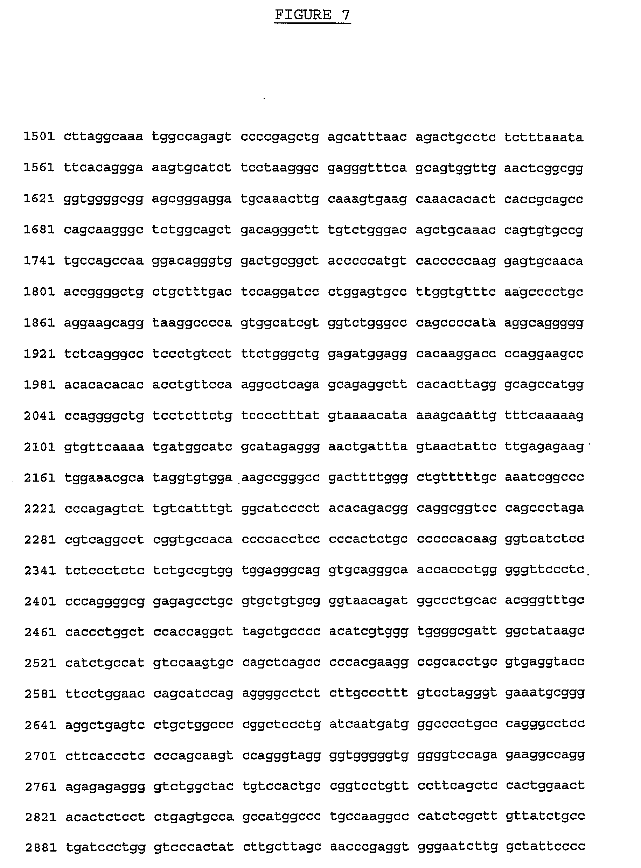

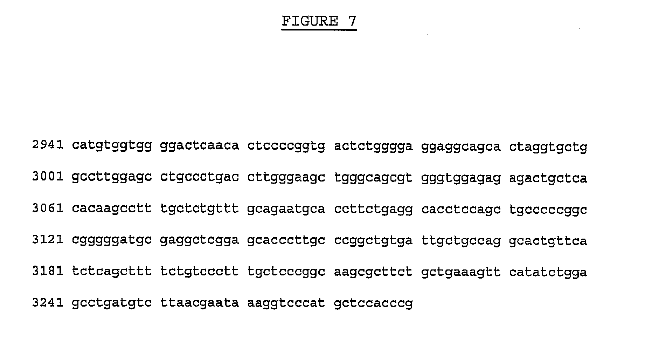

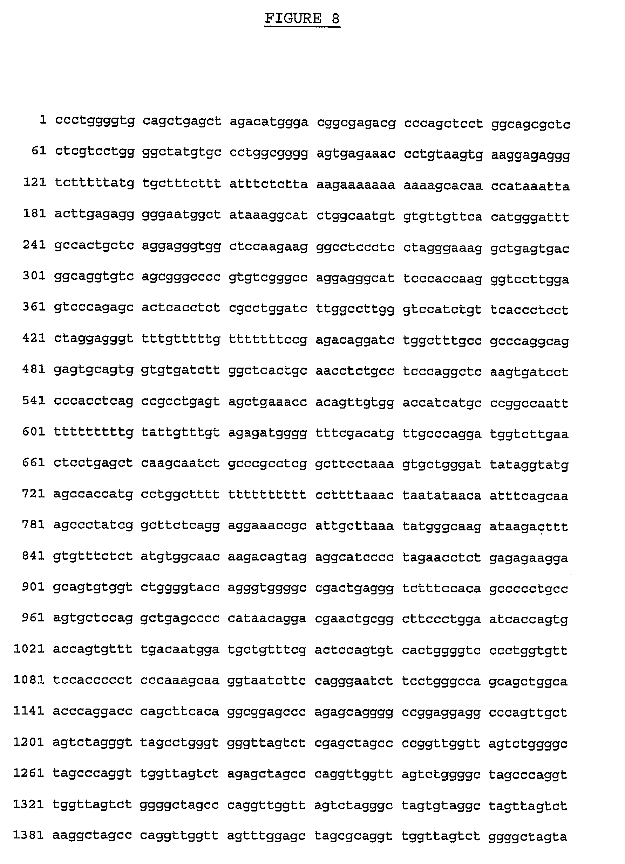

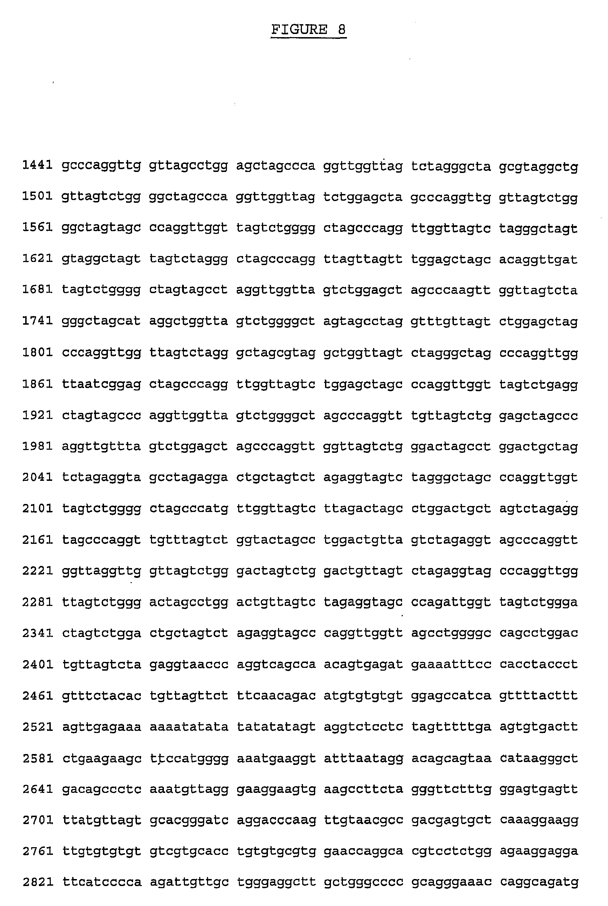

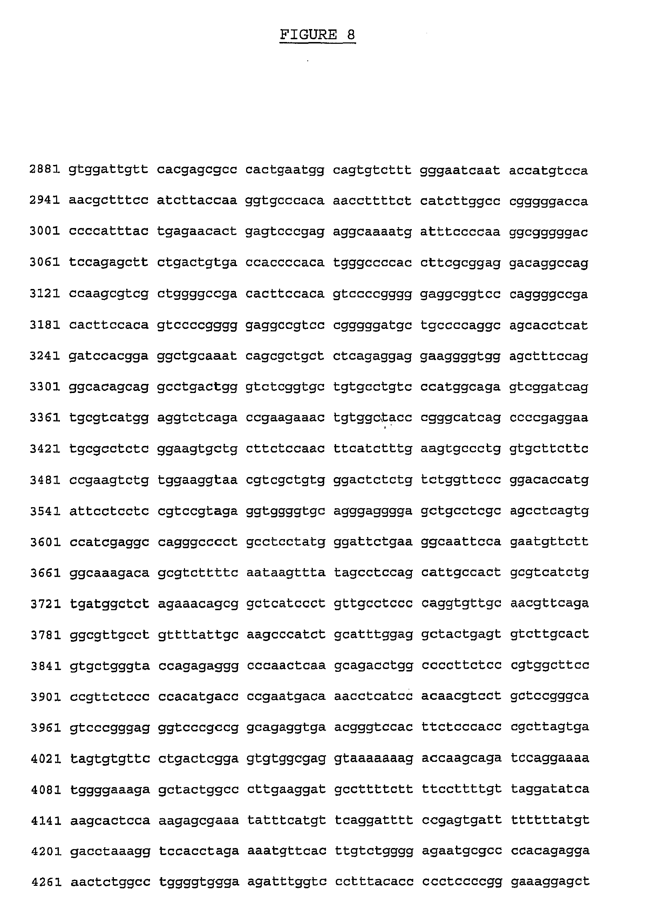

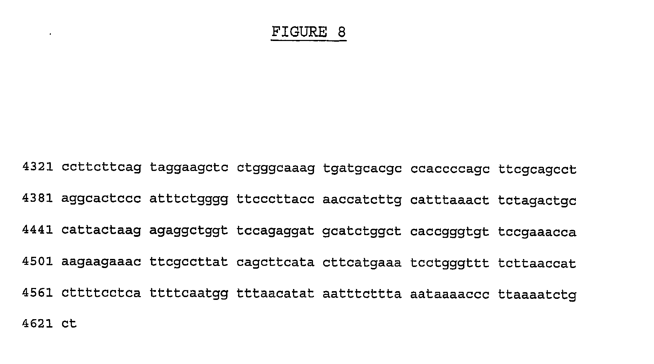

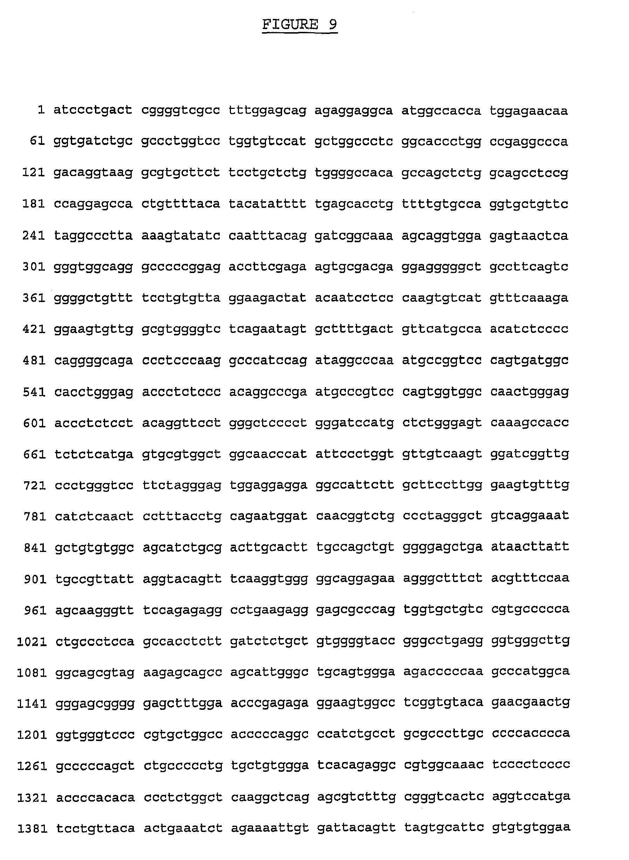

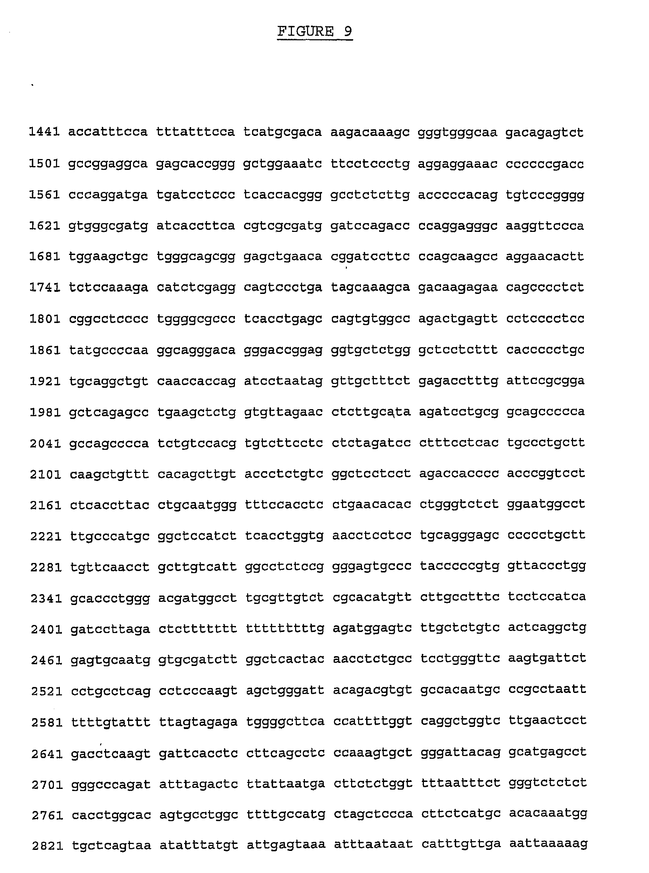

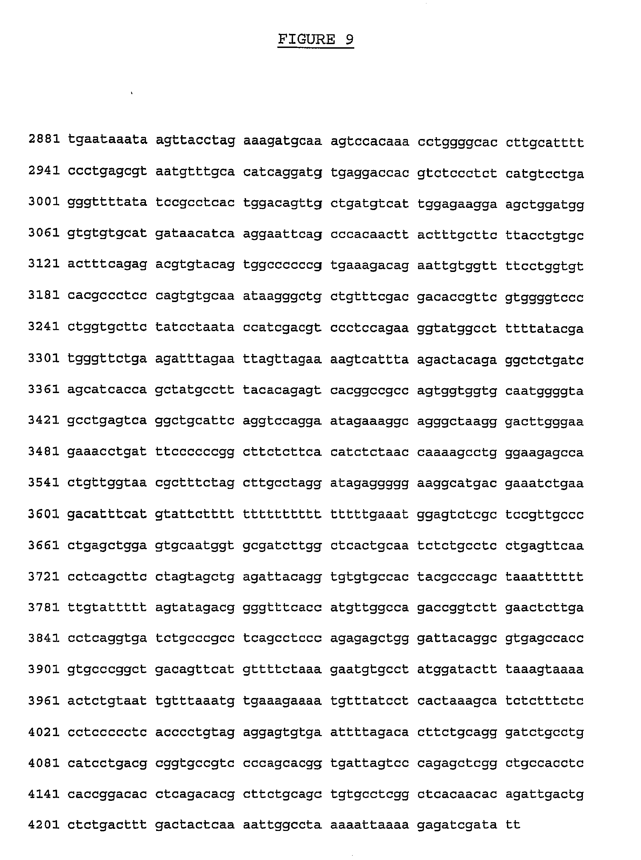

Intestinal trefoil peptides and fragments can be produced by any method known in the art for expression of recombinant proteins. Nucleic acids that encode trefoil peptides (e.g., human intestinal trefoil factor (Figure 4 and 7), human pS2 (Figure 5 and 8), and human spasmolytic polypeptide (Figure 6 and 9) or fragments thereof may be introduced into various cell types or cell-free systems for expression thereby allowing large-scale production, purification, and patient therapy.

Eukaryotic and prokaryotic trefoil peptide expression systems may be generated in which an intestinal trefoil peptide gene sequence is introduced into a plasmid or other vector, which is then used to transform living cells. Constructs in which the intestinal trefoil peptide cDNA contains the entire open reading frame inserted in the correct orientation into an expression plasmid may be used for protein expression. Prokaryotic and eukaryotic expression systems allow for the expression and recovery of intestinal trefoil peptide fusion proteins in which the trefoil peptide is covalently linked to a tag molecule which facilitates identification and/or purification. An enzymatic or chemical cleavage site can be engineered between the trefoil peptide and the tag molecule so that the tag can be removed following purification.

Typical expression vectors contain promoters that direct the synthesis of large amounts of mRNA corresponding to the inserted intestinal trefoil peptide nucleic acid in the plasmid-bearing cells. They may also include a eukaryotic or prokaryotic origin of replication sequence allowing for their autonomous replication within the host organism, sequences that encode genetic traits that allow vector-containing cells to be selected for in the presence of otherwise toxic drugs, and sequences that increase the efficiency with which the synthesized mRNA is translated. Stable long-term vectors may be maintained as freely replicating entities by using regulatory elements of, for example, viruses (e.g., the OriP sequences from the Epstein Barr Virus genome). Cell lines may also be produced that have integrated the vector into the genomic DNA, and in this manner the gene product is produced on a continuous basis.

Expression of foreign sequences in bacteria, such as *Escherichia coli,* requires the insertion of an intestinal trefoil peptide nucleic acid sequence into a bacterial expression vector. Such plasmid vectors contain several elements required for the propagation of the plasmid in bacteria, and for expression of the DNA inserted into the plasmid. Propagation of only plasmid-bearing bacteria is achieved by introducing, into the plasmid, selectable marker-encoding sequences that allow plasmid-bearing bacteria to grow in the presence of otherwise toxic drugs. The plasmid also contains a transcriptional promoter capable of producing large amounts of mRNA from the cloned gene. Such promoters may be (but are not necessarily) inducible promoters that initiate transcription upon induction. The plasmid also preferably contains a polylinker to simplify insertion of the gene in the correct orientation within the vector.

Mammalian cells can also be used to express a trefoil peptide. Stable or transient cell line clones can be made using intestinal trefoil peptide expression vectors to produce the trefoil peptides in a soluble (truncated and tagged) form. Appropriate cell lines include, for example, COS, HEK293T, CHO, or NIH cell lines.

Once the appropriate expression vectors are constructed, they are introduced into an appropriate host cell by transformation techniques, such as, but not limited to, calcium phosphate transfection, DEAE-dextran transfection, electroporation, microinjection, protoplast fusion, or liposome-mediated transfection. The host cells that are transfected with the vectors of this invention may include (but are not limited to) *E*. *coli* or other bacteria, yeast, fungi, insect cells (using, for example, baculoviral vectors for expression in SF9 insect cells), or cells derived from mice, humans, or other animals. *In vitro* expression of trefoil peptides, fusions, or polypeptide fragments encoded by cloned DNA may also be used. Those skilled in the art of molecular biology will understand that a wide variety of expression systems and purification systems may be used to produce recombinant trefoil peptides and fragments thereof. Some of these systems are described, for example, in Ausubel et al. (Current Protocols in Molecular Biology, John Wiley & Sons, New York, NY 2000,

Transgenic plants, plant cells and algae are also particularly useful for generating recombinant intestinal trefoil peptides for use in the methods and compositions of the invention. For example, transgenic tobacco plants or cultured transgenic tobacco plant cells expressing an intestinal trefoil peptide can be created using techniques known in the art (see, for example, U.S. Patent Nos. 5,202,422 and 6,140,075). Transgenic algae expression systems can also be used to produce recombinant intestinal trefoil peptides (see, for example, Chen et al., Curr. Genet. 39:365-370, 2001).

Once a recombinant protein is expressed, it can be isolated from cell lysates using protein purification techniques such as affinity chromatography. Once isolated, the recombinant protein can, if desired, be purified further by e.g., high performance liquid chromatography (HPLC; e.g., see Fisher, Laboratory Techniques In Biochemistry And Molecular Biology, Work and Burdon, Eds., Elsevier, 1980).

Polypeptides of the invention, particularly short intestinal trefoil peptide fragments can also be produced by chemical synthesis using, for example, Merrifield solid phase synthesis, solution phase synthesis, or a combination of both (see, for example, the methods described in Solid Phase Peptide Synthesis, 2nd ed., 1984, The Pierce Chemical Co., Rockford, IL). Optionally, peptide fragments are then be condensed by standard peptide assembly chemistry.

### Dosages

All of the therapeutic agents employed in the compositions of the present invention, including the trefoil peptide component, can be used in the dose ranges currently known and used for these agents. The following are illustrative examples of dose ranges for the active ingredients of the compositions of the invention. Different concentrations of either the trefoil peptide or the other agents may be employed depending on the clinical condition of the patient, the goal of therapy (treatment or prophylaxis), and anticipated duration or severity of the damage for which the agent is being given. Additional considerations in dose selection include: disease etiology, patient age (pediatric, adult, geriatric), general health and comorbidity.

The following examples are intended to illustrate the principle of the present invention and circumstances when trefoil peptide therapy is indicated. The following examples are not intended to be limiting.

### Example 1: Methods for Treating Lesions of the Vaginal Epithelium Caused by Antineoplastic Therapy

Antineoplastic therapy, including chemotherapy and radiotherapy, can damage the vaginal and cervical epithelium. Damage is especially prevalent when wide area radiotherapy is delivered to the abdomen for the treatment of, for example, colorectal, cervical, uterine, ovarian, and prostate cancer. Therapeutic amounts of trefoil peptides can be administered either previous to, concurrent with, or subsequent to antineoplastic therapy and can be delivered, for example, as a vaginal rinse or by vaginal suppository. Trefoil peptide therapy that follows antineoplastic therapy should begin within the first 14 days after the final antineoplastic treatment, preferably within the first 7 days, more preferably within the 3 days, even more preferably within the first day and most preferably, immediately following said final antineoplastic treatment.

Alternatively, trefoil peptide therapy can be administered concurrent to the antineoplastic therapy regime. Effective concurrent therapy consists of trefoil peptide administration within 12 hours of every antineoplastic treatment, preferably within 6 hours of every antineoplastic treatment, even more preferably within 3 hours of every antineoplastic treatment, most preferably, the trefoil peptide is administered simultaneously with every antineoplastic treatment.

Trefoil peptide therapy can also begin prior to initiation of the antineoplastic therapy regime. Pretreatment with a trefoil peptide is prophylactic, thereby mitigating the loss of vaginal or cervical epithelial cells which normally occurs as a consequence of cancer therapy. Preferably, the trefoil peptide is administered for at least one, three, five, or seven days prior to beginning an antineoplastic treatment regime.

The most preferred embodiment of the present method consists of continuous trefoil peptide therapy which is begins with a pretreatment phase, prior to the initiation of antineoplastic therapy, and continues concurrently and subsequently to the cancer therapy.

Vaginal administration of a therapeutically effective amount of a trefoil peptide composition using the method of the present invention is done between once and four times each day, as clinically indicated. Further, clinical indications may necessitate the addition of one or more therapeutic agents, for example, antimicrobials, analgesics, and anti-inflammatories. Additional medicaments can be co-formulated with the trefoil peptides, or may be administered separately.

### Example 2: Treatment of a Vaginal Lesion in Patients Undergoing Episiotomy

The patient is administered an ITF-containing suppository preparation beginning immediately after the repair of the episiotomy incision. The suppository contains therapeutic doses of an ITF and an antibiotic to promote epithelial healing and reduce the likelihood of a post-operative infection. In one example, a vaginal suppository using standard formulation methods will deliver a 100 mg dose of ITF and 100 mg dose of clindamycin. Treatment will continue for the subsequent five days with the patient receiving medication at least once daily. The duration of dosing provides ITF coverage through the entire epithelial restitution period following a vaginal mucosal lesion.

As an alternative to a vaginal suppository, a gel or cream containing the same active ingredients can be used. A more concentrated material, in the form of a gel or paste, can be directly applied to a surgical site using a pledget with a stick applicator.

### Example 3: Treatment of Vaginal Yeast Infections

Yeast *(Candida spp.)* infections often cause damage to the vaginal epithelial layer, resulting in physical discomfort and providing a site for secondary infection. During a course of therapy for severe yeast infections, patients typically receive anti-fungal preparations by vaginal administration. According to this invention, a trefoil peptide, preferable an ITF, can be administered simultaneously with the antifungal treatment. Preferably, the trefoil peptide and the antifungal treatment are contained in the same formulation, however, separate formulations allow for more frequent dosing of either component, as is clinically indicated. Vaginal treatment continues by applying the medication every 6 hours, while awake, for at least the next 72 hours. Therapy is repeated for each subsequent yeast infection. In an alternative dosing regimen, it may be more convenient for these patients to take trefoil peptides less frequently (e.g. every 12 hours) by suppository and, if necessary, supplement the suppository with the cream, ointment, gel, or douche containing higher doses of ITF. Antifungal therapy is generally discontinued once the infection has been eliminated however, trefoil peptide therapy may be continued to induce more rapid healing of the fungal lesion.

## Claims

1. Use of human intestinal trefoil peptide for the manufacture of a medicament for the treatment or prevention of lesions of the vaginal, cervical, or uterine epithelium of a mammal.

2. Use according to claim 1, wherein said intestinal trefoil peptide is spasmolytic polypeptide, pS2, intestinal trefoil factor (ITF), ITF₁₅₋₇₃, ITF₂₁₋₇₃, ITF₁₋₇₂, ITF₁₅₋₇₂, or ITF₂₁₋₇₂.

3. Use according to claim 1, wherein said mammal is a human.

4. Use according to claim 1, wherein said lesion is caused by:
a) the removal of a uterine fibroid;
b) pelvic inflammatory disease; or
c) a bacterial, fungal, or viral infection; preferably said lesion is caused by N. gonorrhea, T. pallidum, Gardnerella spp., Chlamydia spp., Candida albicans, a herpes virus or a papilloma virus.

5. Use according to claim 1, wherein said lesion is caused by a surgical procedure; preferably said surgical procedure is an episiotomy.

6. Use according to claim 1, wherein said lesion:
a) is associated with irritation or pain in said mammal; or
b) is atrophic vaginal mucosa.

7. Use according to one of the claims 1 to 6, wherein said medicament further comprises a second therapeutic agent.

8. Use according to claim 7, wherein said second therapeutic agent is povidone iodine, an anti-inflammatory agent, an antibacterial agent, an anti-fungal agent, an anti-viral agent, an analgesic or a steroid.

9. Use according to claim 8, wherein:
a) said antibacterial agent is a penicillin, a cephalosporin, a tetracycline, or an aminoglycoside;
b) said anti-fungal agent is clindamycin, econaxole, fluconazole, flucytosine, griseofulvin, nystatin, clotrimazole, ketoconazole, enilconazole, itraconazole, butoconazole, tioconazole, or miconazole;
c) said anti-viral agent is acyclovir;
d) said analgesic is lidocaine or benzocaine; or
e) said steroid is triamcinolone, budesonide, or hydrocortisone.

10. Use according to claim 8, wherein said steroid is an estrogen; preferably said estrogen is estradiol.

11. Use according to claim 1, wherein said lesion is a result of antineoplastic therapy; preferably said antineoplastic therapy comprises radiation therapy; more preferably said radiation therapy comprises pelvic radiotherapy.

12. Use according to claim 1, wherein said lesion is a result of antineoplastic therapy which comprises chemotherapy; preferably said chemotherapy is local or systemic.

13. Use according to one of the claims 1 to 12, wherein said medicament is a vaginal suppository, a vaginal rinse, or a vaginal cream.

14. Use according to claim 13, wherein said medicament comprises:
a) microspheres; or
b) a mucoadhesive agent.

15. Use according to one of the claims 1 to 14, wherein said trefoil peptide is in a dimeric form.


SEQUENCE LISTING

<110> The G. I. Company

<120> Methods and Compositions for Treating
  Vaginal, Cervical, and Uterine Epithelial Lesions

<130> 50206/005EP2

<150> PCT/US02/28339
<151> 2002-09-06

<150> US 60/317,657
<151> 2001-09-06

<160> 9

<170> FastSEQ for Windows Version 4.0

<210> 1
<211> 73
<212> PRT
<213> Homo sapien

<400> 1
Met Leu Gly Leu Val Leu Ala Leu Leu Ser Ser Ser Ser Ala Glu Glu
 1               5                  10                  15
Tyr Val Gly Leu Ser Ala Asn Gln Cys Ala Val Pro Ala Lys Asp Arg
            20                  25                  30
Val Asp Cys Gly Tyr Pro His Val Thr Pro Lys Glu Cys Asn Asn Arg
        35                  40                  45
Gly Cys Cys Phe Asp Ser Arg Ile Pro Gly Val Pro Trp Cys Phe Lys
    50                  55                  60
Pro Leu Gln Glu Ala Glu Cys Thr Phe
65                  70

<210> 2
<211> 84
<212> PRT
<213> Homo sapien

<400> 2
Met Ala Thr Met Glu Asn Lys Val Ile Cys Ala Leu Val Leu Val Ser
 1               5                  10                  15
Met Leu Ala Leu Gly Thr Leu Ala Glu Ala Gln Thr Glu Thr Cys Thr
            20                  25                  30
Val Ala Pro Arg Glu Arg Gln Asn Cys Gly Phe Pro Gly Val Thr Pro
        35                  40                  45
Ser Gln Cys Ala Asn Lys Gly Cys Cys Phe Asp Asp Thr Val Arg Gly
    50                  55                  60
Val Pro Trp Cys Phe Tyr Pro Asn Thr Ile Asp Val Pro Pro Glu Glu
65                  70                  75                  80
Glu Cys Glu Phe

<210> 3
<211> 106
<212> PRT
<213> Homo sapien

<400> 3

<210> 4
<211> 222
<212> DNA
<213> Homo sapien

<400> 4
atgctggggc tggtcctggc cttgctgtcc tccagctctg ctgaggagta cgtgggcctg 60
tctgcaaacc agtgtgccgt gccagccaag gacagggtgg actgcggcta cccccatgtc 120
acccccaagg agtgcaacaa ccggggctgc tgctttgact ccaggatccc tggagtgcct 180
tggtgtttca agcccctgca ggaagcagaa tgcaccttct ga                    222

<210> 5
<211> 255
<212> DNA
<213> Homo sapien

<400> 5
atggccacca tggagaacaa ggtgatctgc gccctggtcc tggtgtccat gctggccctc 60
ggcaccctgg ccgaggccca gacagagacg tgtacagtgg ccccccgtga aagacagaat 120
tgtggttttc ctggtgtcac gccctcccag tgtgcaaata agggctgctg tttcgacgac 180
accgttcgtg gggtcccctg gtgcttctat cctaatacca tcgacgtccc tccagaagag 240
gagtgtgaat tttag                                                  255

<210> 6
<211> 390
<212> DNA
<213> Homo sapien

<400> 6
atgggacggc gagacgccca gctcctggca gcgctcctcg tcctggggct atgtgccctg 60
gcggggagtg agaaaccctc cccctgccag tgctccaggc tgagccccca taacaggacg 120
aactgcggct tccctggaat caccagtgac cagtgttttg acaatggatg ctgtttcgac 180
tccagtgtca ctggggtccc ctggtgtttc caccccctcc caaagcaaga gtcggatcag 240
tgcgtcatgg aggtctcaga ccgaagaaac tgtggctacc cgggcatcag ccccgaggaa 300
tgcgcctctc ggaagtgctg cttctccaac ttcatctttg aagtgccctg gtgcttcttc 360
ccgaagtctg tggaagactg ccattactaa                                  390

<210> 7
<211> 3280
<212> DNA
<213> Homo sapien

<400> 7
atgctggggc tggtcctggc cttgctgtcc tccagctctg ctgaggagta cgtgggcctg 60
tgtgagtact gccctgactg ccccggtggc agggtgggcg tgaagggaag ggatccagga 120

taagggggga ttctgcattc atttaataat ggccacctgt cacatataca ctttttcctg 180
cgctagccct ttgaagtggg tctttattgt ccccatttca cagacaagga aaccgaggct 240
cagagaaagt taacaactta tccaaggcag ccctgcccag tctgtgttga aatcagggtt 300
tgagcctgag cccatcccct atgaccccat agccatcttt gctggagatt tctaaattac 360
aatataggtc tttatgcatt gttccacatt tacaaagaaa aaggaaagat gcaggagaaa 420
aaccctgact tcagaacact gtcaataccg gcaggcacaa ggttcattta gccattgcat 480
agcaaccctg ccatggggtg tggctgctcc attaacccaa gtttgaagga atgagggcat 540
ggcttttatc tgggtgtctt ctgagcaggg tcaaaggcag tggttcccga acttgcagcc 600
cattagaatc acctggagag ctttaaaaat cctaatgctt ggggcacacc agttacatca 660
gggcatctcc aggcaagatc caggcctcag ctgttttgtt ttgagatagc cttgctttgt 720
cactcactgc tggagtgcag tggcacaatc tcagctcact gcaacctccg cctcctgggt 780
tcaagcaatt cttgtgcctc ggcttcaagt agctgggatt acaggcatgc accaccatgc 840
ccagctaatt ttttggattt ttagtagaga tggagtttcg ctatgttggc caagctggtc 900
tcaaactcct ggcctcaagt gatcctcctg ccttggcctc ccaaagtgct ggaattacag 960
gtgtaagcca ccatgcccag ccaacgtcag tcatttttaa agctctgcag ctgattccag 1020
tgtgagcgaa gtttggatgc caggaggata agcaattacg gactgggagc aagagaaggg 1080
aatgtaagac actgcacgtg attgccattt tcctaaggaa atactcagtt cgttaatgaa 1140
acgcagtgaa cttctgctgc acatacagac atagaggctt gcctgaaaca tgaaaatatt 1200
ggggactgaa ggatgtcccg ggagggtggg acatgctcaa caattcagga aggggagatg 1260
cagaaaaaag tgaaaagcag gcagcatgcg ttgcaatgat ctctatggcg tgtgcctctc 1320
ctgtcacggt tttcatttaa aacaaagggg caaggttttg ttggtcaaac aatgaagggt 1380
aactttgttt ctgggttcaa gggaccccag attccccagg ggttcctgcc agctggaagg 1440
tacccaggtc cgtatgtgac ttcccgagaa ggtgataaga gcgtgccaag gagaaagaca 1500
cttaggcaaa tggccagagt ccccgagctg agcatttaac agactgcctc tctttaaata 1560
ttcacaggga aagtgcatct tcctaagggc gagggtttca gcagtggttg aactcggcgg 1620
ggtggggcgg agcgggagga tgcaaacttg caaagtgaag caaacacact caccgcagcc 1680
cagcaagggc tctggcagct gacagggctt tgtctgggac agctgcaaac cagtgtgccg 1740
tgccagccaa ggacagggtg gactgcggct acccccatgt cacccccaag gagtgcaaca 1800
accggggctg ctgctttgac tccaggatcc ctggagtgcc ttggtgtttc aagcccctgc 1860
aggaagcagg taaggcccca gtggcatcgt ggtctgggcc cagccccata aggcaggggg 1920
tctcagggcc tccctgtcct ttctgggctg gagatggagg cacaaggacc ccaggaagcc 1980
acacacacac acctgttcca aggcctcaga gcagaggctt cacacttagg gcagccatgg 2040
ccaggggctg tcctcttctg tcccctttat gtaaaacata aaagcaattg tttcaaaaag 2100
gtgttcaaaa tgatggcatc gcatagaggg aactgattta gtaactattc ttgagagaag 2160
tggaaacgca taggtgtgga aagccgggcc gacttttggg ctgtttttgc aaatcggccc 2220
cccagagtct tgtcatttgt ggcatcccct acacagacgg caggcggtcc cagccctaga 2280
cgtcaggcct cggtgccaca ccccacctcc cccactctgc cccccacaag ggtcatctcc 2340
tctccctctc tctgccgtgg tggagggcag gtgcagggca accaccctgg gggttccctc 2400
cccaggggcg gagagcctgc gtgctgtgcg ggtaacagat ggccctgcac acgggtttgc 2460
caccctggct ccaccaggct tagctgcccc acatcgtggg tggggcgatt ggctataagc 2520
catctgccat gtccaagtgc cagctcagcc cccacgaagg ccgcacctgc gtgaggtacc 2580
ttcctggaac cagcatccag aggggcctct cttgcccttt gtcctagggt gaaatgcggg 2640
aggctgagtc ctgctggccc cggctccctg atcaatgatg ggcccctgcc cagggcctcc 2700
cttcaccctc cccagcaagt ccagggtagg ggtgggggtg ggggtccaga gaaggccagg 2760
agagagaggg gtctggctac tgtccactgc cggtcctgtt ccttcagctc cactggaact 2820
acactctcct ctgagtgcca gccatggccc tgccaaggcc catctcgctt gttatctgcc 2880
tgatccctgg gtcccactat cttgcttagc aacccgaggt gggaatcttg gctattcccc 2940
catgtggtgg ggactcaaca ctccccggtg actctgggga ggaggcagca ctaggtgctg 3000
gccttggagc ctgccctgac cttgggaagc tgggcagcgt gggtggagag agactgctca 3060
cacaagcctt tgctctgttt gcagaatgca ccttctgagg cacctccagc tgcccccggc 3120
cgggggatgc gaggctcgga gcacccttgc ccggctgtga ttgctgccag gcactgttca 3180
tctcagcttt tctgtccctt tgctcccggc aagcgcttct gctgaaagtt catatctgga 3240
gcctgatgtc ttaacgaata aaggtcccat gctccacccg 3280

<210> 8
<211> 4623
<212> DNA
<213> Homo sapien

<400> 8
dccctggggt gcagctgagc tagacatggg acggcgagac gcccagctcc tggcagcgct 60

cctcgtcctg gggctatgtg ccctggcggg gagtgagaaa ccctgtaagt gaaggagagg 120
gtctttttat gtgctttctt tatttctctt aaagaaaaaa aaaaagcaca accataaatt 180
aacttgagag ggggaatggc tataaaggca tctggcaatg tgtgttgttc acatgggatt 240
tgccactgct caggagggtg gctccaagaa gggcctccct cctagggaaa ggctgagtga 300
cggcaggtgt cagcgggccc cgtgtcgggc caggagggca ttcccaccaa gggtccttgg 360
agtcccagag cactcacctc tcgcctggat cttggccttg ggtccatctg ttcaccctcc 420
tctaggaggg ttttgttttt gtttttttcc gagacaggat ctggctttgc cgcccaggca 480
ggagtgcagt ggtgtgatct tggctcactg caacctctgc ctcccaggct caagtgatcc 540
tcccacctca gccgcctgag tagctgaaac cacagttgtg gaccatcatg cccggccaat 600
tttttttttt gtattgtttg tagagatggg gtttcgacat gttgcccagg atggtcttga 660
actcctgagc tcaagcaatc tgcccgcctc ggcttcctaa agtgctggga ttataggtat 720
gagccaccat gcctggcttt tttttttttt tccttttaaa ctaatataac aatttcagca 780
aagccctatc ggcttctcag gaggaaaccg cattgcttaa atatgggcaa gataagactt 840
tgtgtttctc tatgtggcaa caagacagta gaggcatccc ctagaacctc tgagagaagg 900
agcagtgtgg tctggggtac cagggtgggg ccgactgagg gtctttccac agccccctgc 960
cagtgctcca ggctgagccc ccataacagg acgaactgcg gcttccctgg aatcaccagt 1020
gaccagtgtt ttgacaatgg atgctgtttc gactccagtg tcactggggt cccctggtgt 1080
ttccaccccc tcccaaagca aggtaatctt ccagggaatc ttcctgggcc agcagctggc 1140
aacccaggac ccagcttcac aggcggagcc cagagcaggg gccggaggag gcccagttgc 1200
tagtctaggg ttagcctggg tgggttagtc tcgagctagc cccggttggt tagtctgggg 1260
ctagcccagg ttggttagtc tagagctagc ccaggttggt tagtctgggg ctagcccagg 1320
ttggttagtc tggggctagc ccaggttggt tagtctaggg ctagtgtagg ctagttagtc 1380
taaggctagc ccaggttggt tagtttggag ctagcgcagg ttggttagtc tggggctagt 1440
agcccaggtt ggttagcctg gagctagccc aggttggtta gtctagggct agcgtaggct 1500
ggttagtctg gggctagccc aggttggtta gtctggagct agcccaggtt ggttagtctg 1560
gggctagtag cccaggttgg ttagtctggg gctagcccag gttggttagt ctagggctag 1620
tgtaggctag ttagtctagg gctagcccag gttagttagt ttggagctag cacaggttga 1680
ttagtctggg gctagtagcc taggttggtt agtctggagc tagcccaagt tggttagtct 1740
agggctagca taggctggtt agtctggggc tagtagccta ggtttgttag tctggagcta 1800
gcccaggttg gttagtctag ggctagcgta ggctggttag tctagggcta gcccaggttg 1860
gttaatcgga gctagcccag gttggttagt ctggagctag cccaggttgg ttagtctgag 1920
gctagtagcc caggttggtt agtctggggc tagcccaggt ttgttagtct ggagctagcc 1980
caggttgttt agtctggagc tagcccaggt tggttagtct gggactagcc tggactgcta 2040
gtctagaggt agcctagagg actgctagtc tagaggtagt ctagggctag cccaggttgg 2100
ttagtctggg gctagcccat gttggttagt cttagactag cctggactgc tagtctagag 2160
gtagcccagg ttgtttagtc tggtactagc ctggactgtt agtctagagg tagcccaggt 2220
tggttaggtt ggttagtctg ggactagtct ggactgttag tctagaggta gcccaggttg 2280
gttagtctgg gactagcctg gactgttagt ctagaggtag cccagattgg ttagtctggg 2340
actagtctgg actgctagtc tagaggtagc ccaggttggt tagcctgggg ccagcctgga 2400
ctgttagtct agaggtaacc caggtcagcc aacagtgaga tgaaaatttc ccacctaccc 2460
tgtttctaca ctgttagttc tttcaacaga catgtgtgtg tggagccatc agttttactt 2520
tagttgagaa aaaaatatat atatatatag taggtctcct ctagtttttg aagtgtgact 2580
tctgaagaag cttccatggg gaaatgaagg tatttaatag gacagcagta acataagggc 2640
tgacagccct caaatgttag ggaaggaagt gaagccttct agggttcttt gggagtgagt 2700
tttatgttag tgcacgggat caggacccaa gttgtaacgc cgacgagtgc tcaaaggaag 2760
gttgtgtgtg tgtcgtgcac ctgtgtgcgt ggaaccaggc acgtcctctg gagaaggagg 2820
attcatcccc aagattgttg ctgggaggct tgctgggccc cgcagggaaa ccaggcagat 2880
ggtggattgt tcacgagcgc ccactgaatg gcagtgtctt tgggaatcaa taccatgtcc 2940
aaacgctttc catcttacca aggtgcccac aaaccttttc tcatcttggc ccgggggacc 3000
accccattta ctgagaacac tgagtcccga gaggcaaaat gatttcccca aggcggggga 3060
ctccagagct tctgactgtg accaccccac atgggcccca ccttcgcgga ggacaggcca 3120
gccaagcgtc gctggggccg acacttccac agtccccggg ggaggcggtc ccaggggccg 3180
acacttccac agtccccggg ggaggccgtc ccgggggatg ctgccccagg cagcacctca 3240
tgatccacgg aggctgcaaa tcagcgctgc tctcagagga ggaaggggtg gagctttcca 3300
gggcacagca ggcctgactg ggtctcggtg ctgtgcctgt cccatggcag agtcggatca 3360
gtgcgtcatg gaggtctcag accgaagaaa ctgtggctac ccgggcatca gccccgagga 3420
atgcgcctct cggaagtgct gcttctccaa cttcatcttt gaagtgccct ggtgcttctt 3480
cccgaagtct gtggaaggta acgtcgctgt gggactctct gtctggttcc cggacaccat 3540
gattcctcct ccgtccgtag aggtggggtg cagggagggg agctgcctcg cagcctcagt 3600
gccatcgagg ccagggcccc tgcctcctat gggattctga aggcaattcc agaatgttct 3660
tggcaaagac agcgtctttt caataagttt atagcctcca gcattgccac tgcgtcatct 3720

gtgatggctc tagaaacagc ggctcatccc tgttgcctcc ccaggtgttg caacgttcag 3780
aggcgttgcc tgttttattg caagcccatc tgcatttgga ggctactgag tgtcttgcac 3840
tgtgctgggt accagagagg gcccaactca agcagacctg gccccttctc ccgtggcttc 3900
cccgttctcc cccacatgac cccgaatgac aaacctcatc cacaacgtcc tgctccgggc 3960
agtcccggga gggtcccgcc ggcagaggtg aacgggtcca cttctcccac ccgcttagtg 4020
atagtgtgtt cctgactcgg agtgtggcga ggtaaaaaaa gaccaagcag atccaggaaa 4080
atggggaaag agctactggc ccttgaagga tgccttttct tttccttttg ttaggatatc 4140
aaagcactcc aaagagcgaa atatttcatg ttcaggattt tccgagtgat tttttttatg 4200
tgacctaaag gtccacctag aaaatgttca cttgtctggg gagaatgcgc cccacagagg 4260
aaactctggc ctggggtggg aagatttggt ccctttacac cccctccccg ggaaaggagc 4320
tccttcttca gtaggaagct cctgggcaaa gtgatgcacg cccaccccag cttcgcagcc 4380
taggcactcc catttctggg gttcccttac caaccatctt gcatttaaac ttctagactg 4440
ccattactaa gagaggctgg ttccagagga tgcatctggc tcaccgggtg ttccgaaacc 4500
aaagaagaaa cttcgcctta tcagcttcat acttcatgaa atcctgggtt ttcttaacca 4560
tcttttcctc attttcaatg gtttaacata taatttcttt aaataaaacc cttaaaatct 4620
gct 4623

<210> 9
<211> 4252
<212> DNA
<213> Homo sapien

<400> 9
atccctgact cggggtcgcc tttggagcag agaggaggca atggccacca tggagaacaa 60
ggtgatctgc gccctggtcc tggtgtccat gctggccctc ggcaccctgg ccgaggccca 120
gacaggtaag gcgtgcttct tcctgctctg tggggccaca gccagctctg gcagcctccg 180
ccaggagcca ctgttttaca tacatatttt tgagcacctg ttttgtgcca ggtgctgttc 240
taggccctta aaagtatatc caatttacag gatcggcaaa agcaggtgga gagtaactca 300
gggtggcagg gcccccggag accttcgaga agtgcgacga ggagggggct gccttcagtc 360
ggggctgttt tcctgtgtta ggaagactat acaatcctcc caagtgtcat gtttcaaaga 420
ggaagtgttg gcgtggggtc tcagaatagt gcttttgact gttcatgcca acatctcccc 480
caggggcaga ccctcccaag gcccatccag ataggcccaa atgccggtcc cagtgatggc 540
cacctgggag accctctccc acaggcccga atgcccgtcc cagtggtggc caactgggag 600
accctctcct acaggttcct gggctcccct gggatccatg ctctgggagt caaagccacc 660
tctctcatga gtgcgtggct ggcaacccat attccctggt gttgtcaagt ggatcggttg 720
ccctgggtcc ttctagggag tggaggagga ggccattctt gcttccttgg gaagtgtttg 780
catctcaact cctttacctg cagaatggat caacggtctg ccctagggct gtcaggaaat 840
gctgtgtggc agcatctgcg acttgcactt tgccagctgt ggggagctga ataacttatt 900
tgccgttatt aggtacagtt tcaaggtggg ggcaggagaa agggctttct acgtttccaa 960
agcaagggtt tccagagagg cctgaagagg gagcgcccag tggtgctgtc cgtgccccca 1020
ctgccctcca gccacctctt gatctctgct gtggggtacc gggcctgagg ggtgggcttg 1080
ggcagcgtag aagagcagcc agcattgggc tgcagtggga agacccccaa gcccatggca 1140
gggagcgggg gagctttgga acccgagaga ggaagtggcc tcggtgtaca gaacgaactg 1200
ggtgggtccc cgtgctggcc acccccaggc ccatctgcct gcgcccttgc ccccacccca 1260
gcccccagct ctgccccctg tgctgtggga tcacagaggc cgtggcaaac tcccctcccc 1320
accccacaca ccctctggct caaggctcag agcgtctttg cgggtcactc aggtccatga 1380
tcctgttaca actgaaatct agaaaattgt gattacagtt tagtgcattc gtgtgtggaa 1440
accatttcca tttatttcca tcatgcgaca aagacaaagc gggtgggcaa gacagagtct 1500
gccggaggca gagcaccggg gctggaaatc ttcctccctg aggaggaaac ccccccgacc 1560
cccaggatga tgatcctccc tcaccacggg gcctctcttg acccccacag tgtcccgggg 1620
gtgggcgatg atcaccttca cgtcgcgatg gatccagacc ccaggagggc aaggttccca 1680
tggaagctgc tgggcagcgg gagctgaaca cggatccttc ccagcaagcc aggaacactt 1740
tctccaaaga catctcgagg cagtccctga tagcaaagca gacaagagaa cagcccctct 1800
cggcctcccc tggggcgccc tcacctgagc cagtgtggcc agactgagtt cctcccctcc 1860
tatgccccaa ggcagggaca gggaccggag ggtgctctgg gctcctcttt caccccctgc 1920
tgcaggctgt caaccaccag atcctaatag gttgctttct gagacctttg attccgcgga 1980
gctcagagcc tgaagctctg gtgttagaac ctcttgcata agatcctgcg gcagccccca 2040
gccagcccca tctgtccacg tgtcttcctc ctctagatcc ctttcctcac tgccctgctt 2100
caagctgttt cacagcttgt accctctgtc ggctcctcct agaccacccc acccggtcct 2160
ctcaccttac ctgcaatggg tttccacctc ctgaacacac ctgggtctct ggaatggcct 2220
ttgcccatgc ggctccatct tcacctggtg aacctcctcc tgcagggagc ccccctgctt 2280

tgttcaacct gcttgtcatt ggcctctccg gggagtgccc tacccccgtg gttaccctgg 2340
gcaccctggg acgatggcct tgcgttgtct cgcacatgtt cttgcctttc tcctccatca 2400
gatccttaga ctcttttttt tttttttttg agatggagtc ttgctctgtc actcaggctg 2460
gagtgcaatg gtgcgatctt ggctcactac aacctctgcc tcctgggttc aagtgattct 2520
cctgcctcag cctcccaagt agctgggatt acagacgtgt gccacaatgc ccgcctaatt 2580
ttttgtattt ttagtagaga tggggcttca ccattttggt caggctggtc ttgaactcct 2640
gacctcaagt gattcacctc cttcagcctc ccaaagtgct gggattacag gcatgagcct 2700
gggcccagat atttagactc ttattaatga cttctctggt tttaatttct gggtctctct 2760
cacctggcac agtgcctggc ttttgccatg ctagctccca cttctcatgc acacaaatgg 2820
tgctcagtaa atatttatgt attgagtaaa atttaataat catttgttga aattaaaaag 2880
tgaataaata agttacctag aaagatgcaa agtccacaaa cctggggcac cttgcatttt 2940
ccctgagcgt aatgtttgca catcaggatg tgaggaccac gtctccctct catgtcctga 3000
gggttttata tccgcctcac tggacagttg ctgatgtcat tggagaagga agctggatgg 3060
gtgtgtgcat gataacatca aggaattcag cccacaactt actttgcttc ttacctgtgc 3120
actttcagag acgtgtacag tggccccccg tgaaagacag aattgtggtt ttcctggtgt 3180
cacgccctcc cagtgtgcaa ataagggctg ctgtttcgac gacaccgttc gtggggtccc 3240
ctggtgcttc tatcctaata ccatcgacgt ccctccagaa ggtatggcct ttttatacga 3300
tgggttctga agatttagaa ttagttagaa aagtcattta agactacaga ggctctgatc 3360
agcatcacca gctatgcctt tacacagagt cacggccgcc agtggtggtg caatggggta 3420
gcctgagtca ggctgcattc aggtccagga atagaaaggc agggctaagg gacttgggaa 3480
gaaacctgat ttccccccgg cttctcttca catctctaac caaaagcctg ggaagagcca 3540
ctgttggtaa cgctttctag cttgcctagg atagaggggg aaggcatgac gaaatctgaa 3600
gacatttcat gtattctttt tttttttttt tttttgaaat ggagtctcgc tccgttgccc 3660
ctgagctgga gtgcaatggt gcgatcttgg ctcactgcaa tctctgcctc ctgagttcaa 3720
cctcagcttc ctagtagctg agattacagg tgtgtgccac tacgcccagc taaatttttt 3780
ttgtattttt agtatagacg gggtttcacc atgttggcca gaccggtctt gaactcttga 3840
cctcaggtga tctgcccgcc tcagcctccc agagagctgg gattacaggc gtgagccacc 3900
gtgcccggct gacagttcat gttttctaaa gaatgtgcct atggatactt taaagtaaaa 3960
actctgtaat tgtttaaatg tgaaagaaaa tgtttatcct cactaaagca tctctttctc 4020
cctccccctc acccctgtag aggagtgtga attttagaca cttctgcagg gatctgcctg 4080
catcctgacg cggtgccgtc cccagcacgg tgattagtcc cagagctcgg ctgccacctc 4140
caccggacac ctcagacacg cttctgcagc tgtgcctcgg ctcacaacac agattgactg 4200
ctctgacttt gactactcaa aattggccta aaaattaaaa gagatcgata tt 4252